# EUROPEAN PATENT SPECIFICATION

(11) **EP 1 613 300 B1**
(45) Date of publication and mention of the grant of the patent: **10.12.2008**
(21) Application number: 04721199.0
(22) Date of filing: 17.03.2004
(51) Int. Cl.: A61K 31/192, A61K 47/14, A61P 19/00, A61P 29/00, A61K 9/06, A61K 47/20

(54) **PHOTOSTABILIZED TOPICAL FORMULATIONS OF KETOPROFEN CONTAINING TWO UV FILTERS**
PHOTOSTABILISIERTE TOPISCHE FORMULIERUNGEN VON KETOPROFEN MIT ZWEI UV-FILTERN
FORMULATIONS TOPIQUES STABILISEES CONTENANT DU KETOPROFENE

(30) Priority: 18.03.2003 IT FI20030070
(43) Date of publication of application: 11.01.2006
(73) Proprietor: BERLIN-CHEMIE AG, 12489 Berlin (DE); Menarini Ricerche S.p.A., 00040 Pomezia (IT)
(72) Inventor: GRÖGER, Karsten, 12587 Berlin (DE); MAGGI, Carlo, Alberto, I-50141 Firenze (IT); MANZINI, Stefano, I-50121 Firenze (IT); SCHMITZ, Reinhard, 10825 Berlin (DE); WIHSMANN, Marc, 15732 Schulzendorf (DE)
(74) Representative: Gervasi, Gemma
(86) International application number: PCT/EP2004/050317
(87) International publication number: WO 2004/082580

(56) References cited:
- EP-A- 1 269 999
- WO-A-92/05768
- WO-A-92/05769
- FR-A- 2 804 024
- US-A- 5 185 373
- US-A1- 2002 028 235
- US-A1- 2002 160 965
- PATENT ABSTRACTS OF JAPAN & JP 60 155111 A (HISAMITSU PHARMACEUTICAL CO. INC.), 15 August 1985 (1985-08-15) cited in the application

## Description

### Field of the invention

Forming the subject of the present invention are formulations containing ketoprofen or its S(+) isomer or mixtures of the two isomers, a combination of two UV filters, and an antioxidant The formulations have very low allergenic and photo-irritant characteristics in regard to the skin, even following upon exposure to sunlight, are well tolerated, and present an excellent penetration of the active principle through the skin.

### State of the art

Topical pharmaceutical formulations containing non-steroidal anti-inflammatory agents (NSAIDs), in particular ketoprofen and dexketoprofen, are described in the majority of the relevant scientific literature as irritant, allergenic, phototoxic, and photo-allergenic (Coz, hristophe J. et al., Contact Dermatitis, 38(5), 245ff (1989), Bosca, F., J. Photochem. Photobiol., 43(3), 1ff (1998)).

Numerous studies have been conducted in the past on the photochemical stablization of formulations containing NSAIDs. An attempt has in fact been made to include NSAIDs, in particular ketoprofen or dexketoprofen in ointments, creams, or gels in such a way as to obtain both physically and chemically stable formulations.

The work of Coz, J. Christophe and F. Bosca were able to demonstrate that some parts of the structure of NSAIDs are inert, whilst others are photosensitive. In the case of ketoprofen, it is the part of the benzophenone that was found to be implicated in photodegradation, whilst the residue of propionic acid was found to be practically insensitive to interaction with light. The specific photo-sensitivity of the diphenyl ketone group is due to its property of initiating the process of oxidation, thanks to the particular stablization of the activated triplet states.

In Photochem. Photobiol., 50(3), 359 (1989), it was seen that four impurities are originated from ketoprofen in aerobic conditions, namely (3-benzoylphenyl)ethane, (3-benzoylphenyl)ethyl hydroperoxide, (3-benzoylphenyl)ethanol and (3-benzoylphenyl)ethanone, whilst in anaerobic conditions only 3-benzoylphenylethane is formed.

The document WO9520387 highlighted the importance of choosing the appropriate moment for application of active substances; in the case of photosensitive substances, these should be applied only at night.

The document JP60155111 describes pharmaceutical formulations for external use, which contain ketoprofen together with a UV filter, chosen in the group of derivatives of *para*-aminobenzoic acid (PABA), anthranilic acid, benzophenone, cinnamic acid, coumarin, salicylic acid, or amino acids and, if necessary, an antioxidant.

Upon more careful analysis, the derivatives of benzophenone appear to be the preferred UV filters, and in particular benzophenone-3, at a concentration of 1 % by weight, with the possible addition of tocopherol as antioxidant, seems to be the only satisfactory one in preserving ketoprofen from photodegradation. Consequently, as may be noted, in no case is a combination, of two UV filters suggested for the formulations described.

In the document FR2804024, the inventors describe topical pharmaceutical forms containing NSAIDs, in particular ketoprofen, protected against photodegradation by means of solar filters chosen in the group: derivatives of cinnamic acid, of dibenzoylmethane, of benzophenone, of *para*-aminobenzoic acid, with the possible presence of an antioxidant chosen from among: butyl hydroxy anisole, *tert*-butyl-*para-*cresol, palmityl ascorbate, or tocopherol. The solutions proposed in the examples described in FR2804024 have not, however, proven completely satisfactory, in particular in the case of ketoprofen. In fact:
- Examples 1 to 9 describe formulations containing ethoxylated esters of *para-*aminobenzoic acid (UV filter) and *tert*-butyl hydroxyanisole (antioxidant), but it has been possible to note that the decomposition of ketoprofen was still present after irradiation (up to 60 % by weight of the initial amount, see Table 1); moreover, there were in any case present problems of phototoxicity of the formulations and the permeability through human skin was considerably reduced if compared to known formulations containing ketoprofen without UV filters (such as, Ketum);
- in Example 10, the use of octyl methoxy cinnamate, together with butyl hydroxy anisole, does not prevent photodegradation of tenoxicam, and problems can be expected regarding the permeability through the skin caused by the large amount of surfactant used;
- a formulation similar to that of Example 11 with sulisobenzone and butyl hydroxy anisole, but using ketoprofen instead of diclofenac, has proved to be phototoxic and has shown a reduced permeability through the skin.

Consequently, pharmaceutical formulations containing ketoprofen prepared on the basis of what is described in JP60155111 and FR280424 are not completely satisfactory. In general, they have proven phototoxic, and moreover it has been noted that the pharmacological properties are sensibly modified when compared with similar well-known formulations containing ketoprofen, but without UV filter, such as, for example, Ketum. On the one hand, the UV filters described cause a change in the chemico-physical characteristics of the formulation (colouring, reduction in viscosity, modification of odour, etc.) and, on the other hand, affect some important pharmacological properties, such as permeability through the skin and the presence of photo-allergic reactions. Normally surfactants are used for stabilizing the formulation and for dissolving the UV filter, but by so doing, permeability through the skin is reduced to a fractional value of what is obtained without said UV filter. Furthermore, the potential of photo-allergenicity increases with the presence of the UV filter. In the case where it is the UV filter that has characteristics of surfactant (for example, the ethoxylated derivatives of *para*-aminobenzoic acid), a considerable reduction in the permeability through the skin has been observed.

In conclusion, what has been described in the foregoing literature would teach the person skilled in the branch how to increase the photostability of pharmaceutical formulations containing ketoprofen, but not, at least up to now, how to prevent the problems of phototoxicity, photo-allergenicity, and reduced permeability through the skin, all of which are problems that still await a proper solution.

### Description of the invention

Surprisingly, it has been found that only formulations containing ketoprofen or its isomer S(+) ketoprofen, together with a mixture of two UV filters, in particular a derivative of cinnamic acid plus phenyl benzimidazol sulphonic acid, and an antioxidant, present, along with an elimination of photodegradation of the active principle, an excellent prevention of the phototoxic and photo-allergenic characteristics, together with an excellent tolerability and an adequate permeation of human skin. Surprisingly, it has also been noted that, although filters of the UV-B type have been used, the formulations described also absorb radiation of the UV-A type (absorption extends to values higher than 360 nm); in this way, it becomes possible to protect both the skin and the ketoprofen also from UV-A radiation, thus reducing the risk of secondary photo-allergenic effects.

In the case of use, as UV filters, of derivatives of benzophenone, of anthranilic acid, of salicylic acid, of *para*-aminobenzoic acid, or of amino-acid-based compounds, as described in JP60155111 or in FR2804024, a photoprotection, even through in general only partial, of the active principle (ketoprofen) was observed, but the system proved in any case to be phototoxic, and the chemico-physical properties were significantly modified. Also the addition of an antioxidant, according to what is explicitly envisaged in JP60155111 and FR2804024, was unable to provide a complete solution to the aforesaid problems.

In the case of octyl methoxy cinnamate as UV filter, the active principle (ketoprofen) proved to be photo-protected, albeit at concentrations of the UV filter higher than the ones used in the mixture of filters; however, notwithstanding this, phototoxicity was observed in many cases. Also formulations containing only phenyl benzimidazol sulphonic acid showed a similar behaviour.

Consequently, mixtures of UV filters have been studied. Different UV filters were added to the derivative of cinnamic acid (for example, derivative of PABA, of benzophenone, or of camphor), but the problems of phototoxicity were not eliminated. In practice, on the basis of our findings, only mixtures of derivatives of cinnamic acid with phenyl benzimidazol sulphonic acid proved satisfactory.

Different compositions containing ketoprofen and a mixture of two UV filters were studied versus the corresponding placebo. In the case of the mixture of octyl methoxy cinnamate and phenyl benzimidazol sulphonic acid as UV filters, the active principle proved photostable and, in the test for determining the phototoxicity *in vitro* (neutral-red-uptake test), there was observed a lower phototoxicity potential. The concentrations of the UV filters in mixture could moreover surprisingly be reduced with respect to the ones necessary when only one of the UV filters was used. It was noted, in fact, that a concentration of 1 % by weight for each of the UV filters - octyl methoxy cinnamate and phenyl benzimidazol sulphonic acid - was sufficient to guarantee the stability of ketoprofen and the maximum reduction of phototoxicity. From the toxicological studies conducted *in vivo*, it was found, for the formulations forming the subject of the present invention, that:
- No skin reactions were attributable to a photo-irritant effect;
- no skin reactions were attributable to photo-allergenic effects;
- no cytotoxic or phototoxic effects were observed; and
- unlike other formulations containing ketoprofen, no photoclastogenic effects were observed (induction of chromosomal aberrations in Chinese-hamster-ovary cell cultures in the presence of UV light).

Furthermore, the problems concerning the permeability of human skin to the active principle in the formulations containing ketoprofen, have been solved.

The present invention thus describes pharmaceutical formulations that contain ketoprofen or its isomers in high percentages but have negligible phototoxic or photo-allergenic effects, high permeability through human skin, and excellent preservation of their pharmacological qualities, if compared to other known ketoprofen-based formulations without UV filters.

The topical formulations based upon the present invention are hence characterized as follows:
- 2-5 % by weight of ketoprofen as a mixture of the two isomers, or 1-2.5 % by weight as single S(+) isomer;
- 0.5-5 % by weight of a derivative of cinnamic acid, preferably octyl-*para*-methoxy cinnamate;
- 0.5-5 % by weight of phenyl benzimidazol sulphonic acid;
- 0.01-0.2 % by weight of a antioxidant; and
- the residual content, up to 100 % by weight, pharmaceutically acceptable excipients, such as adjuvants, vehicles, aromas, stimulants for absorption, etc.

The active principle contained in the aforesaid pharmaceutical formulations is chosen from among ketoprofen, as racemic mixture, the isomer S(+) ketoprofen, or mixtures of the two isomers of ketoprofen. The latter are to be considered both in the form of a free acid and in the form of a pharmaceutically acceptable salt chosen in the group of the salts of sodium, potassium, calcium, magnesium, tromethamine, hydroxyethylamine, di(hydroxyethyl)amine, tri(hydroxyethyl)amine, lysine, arginine, or else in the form of mixtures consisting of a free acid and its salt chosen in the group referred to above.

The percentage of active principle, calculated as free acid in the form of a mixture of the two isomers, contained in the formulation can range from 2 % by weight to 5 % by weight, and in particular from 2.5 % by weight to 5 % by weight; particularly preferred are the ratios between 2.5 % by weight and 4 % by weight. When only the S(+) isomer is considered, the percentage of active principle, calculated as free acid, can range from 1 % by weight to 2.5 % by weight, and in particular from 1.25 % by weight to 2.5 % by weight; particularly preferred are the ratios of 1.25 % by weight to 2 % by weight.

The percentage of UV filter is comprised between 0.5 % by weight and 4% by weight, preferably between 1 % by weight and 3 % by weight. The derivative of cinnamic acid as UV filter is chosen in the group: cinoxate, diethanolamino-*para-*methoxy cinnamate, or among the following esters of cinnamic acid: methyl ester, ethyl ester, propyl ester, isopropyl ester, butyl ester, isoamyl ester, hexyl ester, heptyl ester, *n*-octyl ester, 2-ethyl-hexyl ester, possibly substituted on the aromatic ring with one or two groups chosen from among: hydroxy, methyloxy, ethyloxy, acetylamine; the most preferred one is octyl-methoxy cinnamate (2-ethylhexyl-4-methoxy-cinnamate).

The percentage of antioxidant is comprised between 0.01 % by weight and 0.2 % by weight, preferably between 0.01 % by weight and 0.06 % by weight. As preferred antioxidant is a compound chosen in the group: butyl hydroxy toluene (BHT), butyl hydroxy anisole (BHA), *tert-*butyl-*para-*cresol, tocopherol, tocopherol acetate, tocopherol succinate, ascorbic acid, ascorbyl palmitate, and sodium ascorbate; BHT is the most preferred.

The formulations according to the present invention may moreover contain, as pharmaceutically acceptable excipients, adjuvants, such as water or monoalcohols of the ethanol type, vitamins, amongst which vitamin A and vitamin E are preferred, colouring agents, pigments with colouring effect, inhibitors of the formation of radicals, and preservatives in general, thickeners, plasticizers, humidifiers, aromas - amongst which lavender oil is preferred - , polyols, electrolytes, gelling agents, polar oils and non-polar oils, polymers, copolymers, emulsifiers, stablizers for emulsions, agents for increasing permeability through the skin, etc.

As substances facilitating absorption through the skin, the following are preferred: urea, pyrrolidone, *n*-methyl pyrrolidone, decyl methyl sulphoxide, and sodium lauryl sulphate. Amongst the substances capable of increasing permeability through the skin, also liposomes are to be considered as forming part of the present invention.

As excipients, also active substance commonly used in the cosmetics field can be used, chosen from among: vitamins (amongst which vitamin A and derivatives of vitamin A, vitamin E and derivatives of vitamin E are preferred), vitamin complexes, and coloured or colourless extracts of plants.

Particularly suitable gelling agents are chosen in the group: Carbomers, in particular Carbomer 940 (USP24-NF19; page 2427 (2000)), xanthan gum, carrageenin, acacia gum, guar gum, agar gels, alginates, and methylhydroxy cellulose, carboxymethyl cellulose, hydroxypropyl cellulose, methylhydroxypropyl cellulose, hydroxyethyl cellulose, polyacrylates, polyvinyl alcohol, polyvinylpirrolidone, montmorellonite, etc.

Amongst the humidifiers urea or panthenol may be considered.

As preservatives substances commonly used in pharmacy and cosmetics are preferred, such as esters of hydroxybenzoic acid, or parabens.

According to the excipients used, the topical formulations forming the subject of the invention may hence be in the form of creams, lotions, powders, or gels.

A topical pharmaceutical formulation obtained according to the present invention, expressed as relative quantities of its various components, may, for example, be indicated as follows:
2.5-4 % by weight ketoprofen as mixture of isomers or 1.25-2 % by weight as only S(+) isomer;
0.5-5 % by weight gelling agent;
1-3 % by weight 1 st UV filter (preferably a derivative of cinnamic acid)
1-3 % by weight 2nd UV filter (phenyl benzimidazoyl sulphonic acid);
0-2 % by weight preservative;
0-20 % by weight permeability-enhancers;
0.01-0.06 % by weight BHT;
0-0.1 % by weight sodium EDTA;
0-1 % by weight aromas;
20-90 % by weight ethanol; and
water (as required to reach 100 % by weight).

The pharmaceutical formulations forming the subject of the present invention can be obtained by mixing the components thereof according to the methods well known to a person skilled in the branch.

The pharmaceutical formulations described can be used in the topical treatment of inflammatory pathological conditions or of muscolo-skeletal affections, such as, for instance, myalgias/myosites, and in particular for the topical treatment of pain and inflammation associated to lesions to soft tissue, sprains and distortions, bruises, tendinitis, and venous inflammation.

In addition to the antirheumatic effect, the aforesaid preparations show a photo-allergenic effect that is considerably lower or altogether absent, and this seems extremely important since, as asserted by S. Baudot in Therapie, 53, 137ff (1998), the irritant, allergenic, phototoxic, and photo-allergenic effects of formulations containing NSAIDs lead to hospitalization in 10% of the subjects treated.

For the purposes of the present invention, the following specifications must be borne in mind:
- by "benzophenones" are meant compounds containing the structure of benzophenone and known by the INCl names from benzophenone-1 to benzophenone-11 (amongst which, for example, benzophenone-3 (2-hydroxy-4-methoxy-benzophenone), or benzophenone-4 (2-hydroxy-4-methoxy-benzophenone-5-sulphonic acid);
- by "derivatives of *para*-aminobenzoic acid" are meant compounds in the group: ethyl, propyl, butyl, isopropyl, and monoglyceryl esters of *para*-aminobenzoic acid, ethyl and amyl esters of *para*-dimethylaminobenzoic acid, ethyl and amyl esters of *para*-diethylaminobenzoic acid, and polyethoxyethyl ester of 4-*bis*(polyethoxy)-*para-*aminobenzoic acid;
- by "phenyl benzimidazol sulphonic acid" is meant preferably 2-phenyl-benzimidazol-5-sulphonic acid known as Ensulizol in USP.

Appearing below are some non-limiting examples of topical pharmaceutical formulations obtained according to the present invention. All the ingredients are indicated according to weight percent, unless otherwise specified.

### Example 1

The formulation was made by mixing the individual ingredients according to the techniques known in the state of the art.

Ketoprofen 2.5 g, hydroxypropylmethyl cellulose 2.1 g, ethanol 52.0 g, lavender oil 0.1 g, octyl methoxy cinnamate 3.1 g, phenyl benzimidazol sulphonic acid 1.0 g, Trometamol to reach pH 5.4, sodium EDTA 0.003 g, diethyleneglycol-monomethylether 13.0 g, BHT 0.01 g, water as required to reach 100 g.

Preparation: The water was heated to 30°C, and hydroxypropylmethyl cellulose was added rapidly and under stirring. The mixture was kept under stirring for 30 min at 30°C in order to imbibe the hydroxypropylmethyl cellulose, after which ketoprofen, lavender oil, octyl methoxy cinnamate, phenyl benzimidazol sulphonic acid/ Trometamol, and BHT were added. The mixture was stirred slowly until the temperature dropped below 30°C, and diethyleneglycol monomethylether, ethanol and other missing excipients were added. The product was stirred to obtain a homogeneous dispersion and cooled under stirring, homogenizing for 60 min.

### Example 2

Ketoprofen 2.5 g, hydroxypropylmethyl cellulose 2.5 g, ethanol 52.0 g, lavender oil 0.1 g, octyl methoxy cinnamate 1.0 g, phenyl benzimidazol sulphonic acid 1.0 g, Trometamol to reach pH 5.5, diethyleneglycol-monomethylether 15.0 g, sodium EDTA 0.003 g, BHT 0.02 g, water as required to reach 100 g.

Preparation: in a way similar to Example 1.

### Example 3

S(+) ketoprofen 1.0 g, hydroxypropylmethyl cellulose 2.0 g, ethanol 55.0 g, glycerine 3.0 g, lavender oil 0.11 g, octyl methoxy cinnamate 2.0 g , phenyl benzimidazol sulphonic acid 1.0 g, tromethamine to reach pH 5.4, sodium EDTA 0.003 g, diethyleneglycol-monomethylether 10.0 g, BHT 0.02 g, water as required to reach 100 g.

Preparation: in a way similar to Example 1.

### Example 4

Ketoprofen 2.5 g, hydroxypropylmethylcellulose 2.0 g, ethanol 64.14 g, lavender oil 0.2 g, octyl methoxy cinnamate 1.0 g, phenyl benzimidazol sulphonic acid 1.0 g, Trometamol to reach pH 5.4, sodium EDTA 0.003 g, BHT 0.06 g, water as required to reach 100 g.

Preparation: The water was heated to 40°C, and hydroxypropylmethyl cellulose was added rapidly:and under stirring. The mixture was kept under shirring for 30 min at 40°C in order to imbibe the hydroxypropylmethyl cellulose. In a second container, ketoprofen and all the other components were dissolved in ethanol. The aqueous gel was brought to 30°C, and the ethanol solution was added under stirring. The product was stirred until a homogeneous dispersion was obtained, and then cooled under stirring, homogenizing for 60 min.

### Example 5

Ketoprofen 2.5 g, hydroxypropylcellulose1.0 g, Carbomer 940 (USP) 1.0 g, ethanol 54.5 g, lavender oil 0.2 g, octyl methoxy cinnamate 2.0 g, phenyl benzimidazol sulphonic acid 1.0 g, Trometamol to reach pH 5.4, diethyleneglycol-monomethylether 10.0 g, BHT 0.05 g, sodium EDTA 0.003 g, urea 3.0 g, aminomethyl propanol 0.1 g, water as required to reach 100 g.

Preparation: The procedure was as for Example 4, with the difference that the mixture of hydroxypropylcellulose and Carbomer was imbibed in an aqueous solution containing aminomethyl propanol and urea, whilst all the other components were dissolved in ethanol/diethylene glycol-methyl ether.

### Example 6

Ketoprofen 2.5 g, hydroxypropylcellulose 1.0 g, hydroxypropylmethyl cellulose 1.0 g, ethanol 44.0 g, 2-propanol 20.0 g, lavender oil 0.2 g, octyl methoxy cinnamate 1.0 g, phenyl benzimidazol sulphonic acid 3.0 g, Trometamol to reach pH 5.4, sodium EDTA 0.003 g, BHT 0.05 g, water as required to reach 100 g.

Preparation: in a way similar to what is described in Example 5.

### Example 7

Ketoprofen 2.5 g, hydroxypropyl cellulose2.0 g, ethanol 88.0 g, lavender oil 0.2 g, octyl methoxy cinnamate 1.0 g, phenyl benzimidazol sulphonic acid 1.0 g, Trometamol to reach pH 5.4, BHT 0.05 g, water as required to reach 100 g. Preparation: in a way similar to what is described in Example 1.

### Example 8

Ketoprofen 2.5 g, hydroxypropyl cellulose 2.0 g, ethanol 90.2 g, lavender oil 0.2 g, octyl methoxy cinnamate 1.0 g, phenyl benzimidazol sulphonic acid 1.0 g, Trometamol to reach pH 5.4, BHT 0.05 g, pyrrolidone 3.0g, water as required to reach 100 g. Preparation: All the components with the exception of hydroxypropylcellulose were added to the ethanol and were stirred gently until a clear solution was obtained (30 min). Hydroxypropylcellulose was then added; and the product was stirred vigorously for 2 hours.

### Examples for comparison

### Example A: "PABA only"

Ethanol 40 g, ketoprofen, 2.5 g, Carbomer 940 1.8 g, BHA 0.05 g, lavender oil 0.1 g, polyethoxyethylester of 4-bis(polyethoxy)-*para*-aminobenzoic acid 4.0 g, triethanolamine to reach pH 6, water as required to reach 100 g.

### Example B: "OMC only"

Ethanol 40 g, ketoprofen, 2.5 g, Garbomer 940 2.0 g, BHA 0.05 g, lavender oil 0.1 g, octyl methoxy cinnamate 3.0 g, glycolethylene monomethylether 15.0 g, Cremophor RH4010.0 g, Trometamol to reach pH 5, water as required to reach 100 g.

### Pharmacological tests

Photodegradation: after irradiation with a Schott WG320/1.5 mm xenon lamp, with 100 klux/h and 75 Wh/m. Table 1 gives the percentage of ketoprofen degraded after irradiation for one hour with approximately 10 J/cm² in two preparations of the present invention, as compared to formulations according to the prior art.

**Table 1**

| Formulation | Degradation of ketoprofen [%] |
|---|---|
| FR2804024 Example 1 | 40 |
| FR2804024 Example 7 | 55 |
| FR2804024 Example 8 | 60 |
| Example 1 of this invention | 1.0 |
| Example 2 of this invention | 2.5 |
| System with ketoprofen/benzophenone-4/ ascorbic acid (JP60155111) | 8 |

Table 2 gives the percentage of ketoprofen remaining after irradiation in formulations protected with PABA or methoxy cinnamate (OMC) either alone or in mixture with phenyl benzimidazol sulphonic acid (PBISS).

**Table 2**

| Formulations | time [min] | | | | |
|---|---|---|---|---|---|
| | 0 | 10 | 20 | 30 | 60 |
| Example A | 100 | 87.9 | 73.9 | 69.3 | 41.6 |
| Example B | 100 | 100 | 100 | 100 | 99.6 |
| Example 2 of this invention | 100 | 99.9 | 100 | 100 | 99.9 |

### Phototoxicity:

A commercially available sun lotion with a factor of protection 8, containing a UV filter of an inorganic type (TiO₂/ZnO), was applied on the skin of the back of two groups of 10 healthy human subjects.

The formulation described in Example 1 was applied to the first of the groups of 10 subjects (group I), who had already been treated with 2 mg/cm of sun cream, on their backs (skin type II). Applied on the skin of the second group of 10 subjects (group II, skin type II) was a formulation with all the ingredients of Example 1 except for octyl methoxy cinnamate. The subjects underwent irradiation with a Schott WG320/1.5 mm xenon lamp, with 100 klux/h and 75 Wh/m in 5 doses, and were examined after 24 h. No reddening of the skin was observed after 24 h in group I; in group II, the skin of 3 subjects was not reddened, whilst 4 subjects showed a slight reddening, and 3 subjects presented medium reddening of the skin.

### Photo-allergenic reaction:

Different formulations containing ketoprofen, a UV filter, and possibly an agent for enhancing the permeability through the skin were compared with the respective placebos, and in all cases the phototoxicity was examined *in vitro* (neutral-red-uptake test). The test was conducted according to the standard procedure COLIPA. In the tests conducted, the placebos in no case proved phototoxic.

Using the formulations containing the active principle, an antioxidant and, as UV filter, octyl methoxy cinnamate, and phenyl benzoimidazol sulphonic acid, no phototoxic effects were observed. In the case of use of other UV filters (such as benzophenone-3, benzophenone-4, derivatives of salicylic acid), the formulations were in all cases phototoxic even when the active principle was kept quite stable during irradiation.

In Table 3, the results obtained with two formulations described in the examples are compared with the results obtained with formulations prepared as described in FR2804024 and JP60155111.

From these results it is possible to deduce that formulations with ketoprofen/cinnamate-phenyl benzimidazol sulphonic acid/BHT do not lead to photo-allergenic reactions.

**Table 3**

| Formulation | Phototoxicity |
|---|---|
| FR2804024 Example 2 | +++ |
| FR2804024 Example 4 | +++ |
| FR2804024 Example 9 | ++ |
| Example 2 of this invention | - |
| Example 3 of this invention | - |
| Example B | ++ |
| System with ketoprofen/benzophenone-4/ ascorbic acid (JP60155111) | ++ |

### Permeability:

Table 4 shows the results on the permeability through human skin of various formulations, all performed in comparison with similar formulations without the UV filter and the antioxidant.

**Table 4**

| Formulations | Permeability (in comparison to the formulation without UV filter) |
|---|---|
| FR2804024 Example 3 | --- |
| FR2804024 Example 5 | --- |
| FR2804024 Example 8 | -- |
| Example B | -- |
| Example 2 of this invention | ++ |
| Example 5 of this invention | +++ |
| Example 8 of this invention | +++ |
| System with ketoprofen/benzophenone-4/ α-tocopherol (JP60155111) | + |

## Claims

1. A topical pharmaceutical composition containing:
a) a non-steroidal anti-inflammatory agent chosen from among: ketoprofen, as racemic mixture, the isomer S(+) ketoprofen, or mixtures of the two isomers of ketoprofen; these are to be considered both in the form of free acid and in the form of a pharmaceutically acceptable salt chosen in the group of the salts of sodium, potassium, calcium, magnesium, tromethamine, hydroxyethylamine, di(hydroxyethyl)amine, tri(hydroxyethyl)amine, lysine, arginine or else in the form of mixtures consisting of a free acid and its salt, chosen in the group referred to above;
b) a derivative of cinnamic acid, as UV filter, chosen in the group: a) cinoxate, diethanolamino-*para*-methoxy cinnamate b) ester of cinnamic acid chosen in the group: methyl ester, ethyl ester, propyl ester, isopropyl ester, butyl ester, isobutyl ester, amyl ester isoamyl ester, hexyl ester, heptyl ester, *n*-octyl ester, and 2-ethyl-hexyl ester, optionally substituted on the aromatic ring with one or two groups chosen from among: hydroxy, methoxy, ethoxy, acetylamine;
c) 2-phenyl benzimidazol-5-sulphonic acid as additional UV filter;
d) an antioxidant, chosen in the group: butyl hydroxy toluene (BHT) and butyl hydroxy anisole (BHA);
together with pharmaceutically acceptable excipients.

2. The topical pharmaceutical composition according to Claim 1, in which the derivative of cinnamic acid as UV filter is the 2-ethyl-hexyl ester of 4-methoxy cinnamic acid (octyl methoxy cinnamate).

3. The topical pharmaceutical composition according to Claims 1 and 2, in which the concentration of ketoprofen, calculated as free acid, ranges from 2 % by weight to 5 % by weight when it is the racemic form or the mixture of isomers, and from 1 % by weight to 2.5 % by weight when it is the S(+) isomer.

4. The topical pharmaceutical composition according to Claim 3, in which the concentration of ketoprofen, calculated as free acid, ranges from 2.5 % by weight to 5 % by weight when it is the racemic form, or from 1.25 % by weight to 2.5 % by weight when it is the S(+) isomer.

5. The topical pharmaceutical composition according to Claim 4, in which the concentration of ketoprofen, calculated as free acid, ranges from 2.5 % by weight to 4 % by weight when it is the racemic form, or from 1.25 % by weight to 2 % by weight when it is the S(+) isomer.

6. The topical pharmaceutical composition according to Claim 2, in which the concentration of octyl methoxy cinnamate UV filter ranges from 0.5 % by weight to 5 % by weight.

7. The topical pharmaceutical composition according to Claim 6, in which the concentration of the octyl methoxy cinnamate UV filter ranges from 1 % by weight to 3 % by weight.

8. The topical pharmaceutical composition according to Claim 1, in which the concentration of the 2-phenyl-benzimidazol-5-sulphonic acid UV filter ranges from 0.5 % by weight to 5 % by weight.

9. The topical pharmaceutical composition according to Claim 8, in which the concentration of the 2-phenyl-benzimidazol-5-sulphonic acid UV filter ranges from 1 % by weight to 3 % by weight.

10. The topical pharmaceutical composition according to Claim 1, in which the oxidant is butyl hydroxy toluene (BHT), and its concentration ranges from 0.01 % by weight to 0.2 % by weight.

11. The topical pharmaceutical composition according to Claim 10, in which the concentration of the antioxidant BHT ranges from 0.01 % by weight to 0.06 % by weight.

12. The pharmaceutical compositions according to any one of Claims 1 to 11, in which the agent for enhancing permeability through the skin possibly used is a substance chosen in the group: urea, pyrrolidone, *N*-methyl pyrrolidine, decylmethyl sulphoxide, sodium lauryl sulphate, and dimethyl sulphoxide.

13. The pharmaceutical composition according to any one of Claims 1 to 12, in which lavender oil is used as aroma.

14. The pharmaceutical compositions according to any one of Claims 1 to 13, in the form of creams, gels, lotions, sprays or powders, in which the non-steroidal anti-inflammatory agent is protected from photodegradation, and the composition does not have phototoxic and photo-allergenic residual effects following upon application on the skin.

15. The topical pharmaceutical composition according to Claim 1, which contains: 2.5-4 % by weight ketoprofen as mixture of isomers or 1.25-2 % by weight as S(+) isomer alone; 0.5-5 % by weight gelling agent; 1-3 % by weight 1st UV filter (preferably, a derivative of cinnamic acid); 1-3 % by weight 2nd UV filter (phenyl benzimidazoyl sulphonic acid); 0-2 % by weight preservative; 0-20 % by weight permeability-enhancing agent; 0.01-0.06 % by weight BHT; 0-0.1 % by weight sodium EDTA; 0-1 % by weight aromas; 20-90 % by weight ethanol; water as required to reach 100 % by weight.

16. Use of octyl methoxy cinnamate in combination with 2-phenyl benzimidazol-5-sulphonic acid and BHT for the preparation of pharmaceutical compositions according to claim 1, for the topical treatment of inflammatory pathological conditions and pain.

17. Pharmaceutical formulations according to Claim 1 for the topical treatment of pain and of inflammation associated to lesions to soft tissue, sprains and distortions, bruises, tendinitis, and venous inflammation.

## Patentansprüche

1. Topische pharmazeutische Zusammensetzung enthaltend:
a) ein nicht-steroides entzündungshemmendes Mittel, ausgewählt aus Ketoprofen als racemische Mischung, dem Isomer S(+)Ketoprofen oder Mischungen der zwei Isomere von Ketoprofen; diese können in Form der freien Säure und in Form eines pharmazeutisch akzeptablen Salzes, ausgewählt aus der Gruppe der Salze von Natrium, Kalium, Calcium, Magnesium, Tromethamin, Hydroxyethylamin, Di(hydroxyethyl)amin, Tri(hydroxyethyl)amin, Lysin, Arginin oder andernfalls in Form von Mischungen vorliegen, die aus einer freien Säure und ihrem Salz, das ausgewählt wird aus der oben genannten Gruppe, besteht;
b) ein Derivat der Zimtsäure als UV-Filter, ausgewählt aus der Gruppe: a) Cinoxat, Diethanolamino-paramethoxycinnamat, b) Ester der Zimtsäure, ausgewählt aus der Gruppe: Methylester, Ethylester, Propylester, Isopropylester, Butylester, Isobutylester, Amylester, Isoamylester, Hexylester, Heptylester, n-Octylester und 2-Ethylhexylester, gegebenenfalls am aromatischen Ring mit einer oder mehreren Gruppen, ausgewählt aus: Hydroxy, Methoxy, Ethoxy, Acetylamin substituiert;
c) 2-Phenylbenzimidazol-5-sulfonsäure als zusätzlichen UV-Filter;
d) ein Antioxidans, ausgewählt aus der Gruppe: Butylhydroxytoluol (BHT) und Butylhydroxyanisol (BHA);
zusammen mit pharmazeutisch akzeptablen Hilfsstoffen.

2. Topische pharmazeutische Zusammensetzung gemäß Anspruch 1, in welcher das als UV-Filter verwendete Zimtsäurederivat der 2-Ethylhexylester von 4-Methoxyzimtsäure (Octylmethoxycinnamat) ist.

3. Topische pharmazeutische Zusammensetzung gemäß den Ansprüchen 1 und 2, in welcher die Konzentration an Ketoprofen, berechnet als freie Säure, 2 Gew.% bis 5 Gew.% beträgt, wenn es in der racemischen Form oder der Mischung der Isomere vorliegt, und 1 Gew.% bis 2,5 Gew.%, wenn es das S(+)Isomer ist.

4. Topische pharmazeutische Zusammensetzung gemäß Anspruch 3, in welcher die Konzentration an Ketoprofen, berechnet als freie Säure, 2,5 Gew.% bis 5 Gew.% beträgt, wenn es in der racemischen Form vorliegt, oder 1,25 Gew.% bis 2,5 Gew.%, wenn es als S(+)Isomer vorliegt.

5. Topische pharmazeutische Zusammensetzung gemäß Anspruch 4, in welcher die Konzentration an Ketoprofen, berechnet als freie Säure, 2,5 Gew.% bis 4 Gew.% beträgt, wenn es in der racemischen Form vorliegt, oder 1,25 Gew.% bis 2 Gew.%, wenn es als S(+)Isomer vorliegt.

6. Topische pharmazeutische Zusammensetzung gemäß Anspruch 2, in welcher die Konzentration des Octylmethoxycinnamat-UV-Filters 0,5 Gew.% bis 5 Gew.% beträgt.

7. Topische pharmazeutische Zusammensetzung gemäß Anspruch 6, in welcher die Konzentration des Octylmethoxycinnamat-UV-Filters 1 Gew.% bis 3 Gew.% beträgt.

8. Topische pharmazeutische Zusammensetzung gemäß Anspruch 1, in welcher die Konzentration des 2-Phenylbenzimidazol-5-sulfonsäure-UV-Filters 0,5 Gew.% bis 5 Gew.% beträgt.

9. Topische pharmazeutische Zusammensetzung gemäß Anspruch 8, in welcher die Konzentration des 2-Phenylbenzimidazol-5-sulfonsäure-UV-Filters 1 Gew.% bis 3 Gew.% beträgt.

10. Topische pharmazeutische Zusammensetzung gemäß Anspruch 1, in welcher das Oxidans Butylhydroxytoluol (BHT) ist und dessen Konzentration 0,01 Gew.% bis 0,2 Gew.% beträgt.

11. Topische pharmazeutische Zusammensetzung gemäß Anspruch 10, in welcher die Konzentration des Antioxidans BHT 0,01 Gew.% bis 0,06 Gew.% beträgt.

12. Pharmazeutische Zusammensetzung gemäß einem der Ansprüche 1 bis 11, in welcher das gegebenenfalls verwendete Mittel zur Erhöhung der Hautpermeabilität eine Substanz ausgewählt aus der Gruppe: Harnstoff, Pyrrolidon, N-Methylpyrrolidin, Decylmethylsulfoxid, Natriumlaurylsulfat und Dimethylsulfoxid ist.

13. Pharmazeutische Zusammensetzung gemäß einem der Ansprüche 1 bis 12, in welcher Lavendelöl als Duftstoff verwendet wird.

14. Pharmazeutische Zusammensetzung gemäß einem der Ansprüche 1 bis 13 in Form von Cremes, Gelen, Lotionen, Sprays oder Pudern, wobei das nicht-steroide entzündungshemmende Mittel vor Fotoabbau geschützt ist und die Zusammensetzung nach dem Aufbringen auf die Haut keine Effekte aufgrund von fototoxischen und fotoallergenen Rückständen aufweist.

15. Topische pharmazeutische Zusammensetzung gemäß Anspruch 1, enthaltend: 2,5 bis 4 Gew.% Ketoprofen als Isomerenmischung oder 1,25 bis 2 Gew.% des S(+)Isomers alleine; 0,5 bis 5 Gew.% Geliermittel; 1 bis 3 Gew.% eines ersten UV-Filters (bevorzugt ein Derivat der Zimtsäure); 1 bis 3 Gew.% eines zweiten UV-Filters (Phenylbenzimidazolylsulfonsäure); 0 bis 2 Gew.% Konservierungsmittel; 0 bis 20 Gew.% eines Permeabilitätsverstärkers; 0,01 bis 0,06 Gew.% BHT; 0 bis 0,1 Gew.% Natrium-EDTA; 0 bis 1 Gew.% Duftstoffe; 20 bis 90 Gew.% Ethanol; Wasser soviel wie benötigt, um 100 Gew.% zu erreichen.

16. Verwendung von Octylmethoxycinnamat in Kombination mit 2-Phenyl-benzimidazol-5-sulfonsäure und BHT zur Herstellung von pharmazeutischen Zusammensetzungen gemäß Anspruch 1 zur topischen Behandlung von entzündlichen pathologischen Zuständen und Schmerzen.

17. Pharmazeutische Formulierungen gemäß Anspruch 1 für die topische Behandlung von Schmerzen und von Entzündungen, die im Zusammenhang mit Schädigungen der Weichgewebe, Verstauchungen und Zerrungen, Blutergüssen, Sehnenentzündung und venöser Entzündung stehen.

## Revendications

1. Composition pharmaceutique topique comprenant :
a) un agent anti-inflammatoire non stéroïdien choisi parmi le kétoprofène, en tant que mélange racémique, l'isomère S(+) kétoprofène, ou des mélanges des deux isomères du kétoprofène ; incluant à la fois la forme acide libre et la forme d'un sel pharmaceutiquement acceptable choisi dans le groupe constitué par les sels de sodium, potassium, calcium, magnésium, throméthamine, hydroxyéthylamine, di(hydroxyéthyl)amine, tri(hydroxyéthyl)amine, lysine, arginine ou sous la forme de mélanges comprenant un acide libre et son sel, choisis dans le groupe ci-dessus ;
b) un dérivé de l'acide cinnamique, en tant que filtre UV, choisi dans le groupe constitué par a) le cinoxate, le diéthanolamino-para-méthoxycinnamate, b) l'ester de l'acide cinnamique choisi dans le groupe constitué par l'ester méthylique, l'ester éthylique, l'ester propylique, l'ester isopropylique, l'ester butylique, l'ester isobutylique, l'ester amylique, l'ester isoamylique, l'ester hexylique, l'ester heptylique, l'ester n-octylique, et l'ester 2-éthylhexylique, éventuellement substitué sur le cycle aromatique par un ou deux groupes choisis parmi l'hydroxy, le méthoxy, l'éthoxy et l'acétylamine ;
c) l'acide 2-phénylbenzimidazol-5-sulfonique en tant que filtre UV additionnel ;
d) un antioxydant choisi dans le groupe constitué par le butylhydroxytoluène (BHT) et le butylhydroxyanisole (BHA) ;
avec des excipients pharmaceutiques acceptables.

2. Composition pharmaceutique topique selon la revendication 1, dans laquelle le dérivé de l'acide cinnamique en tant que filtre UV est l'ester 2-éthylhexylique de l'acide 4-méthoxycinnamique (méthoxycinnamate d'octyle).

3. Composition pharmaceutique topique selon la revendication 1 ou 2, dans laquelle la concentration en kétoprofène, calculée en tant qu'acide libre, est comprise dans la plage allant de 2 % en poids à 5 % en poids lorsque c'est la forme racémique ou le mélange d'isomères, et de 1 % en poids à 2,5 % en poids lorsque c'est l'isomère S(+).

4. Composition pharmaceutique topique selon la revendication 3, dans laquelle la concentration en kétoprofène, calculée en tant qu'acide libre, est comprise dans la plage allant de 2,5 % en poids à 5 % en poids lorsque c'est la forme racémique, et de 1,25 % en poids à 2,5 % en poids lorsque c'est l'isomère S(+).

5. Composition pharmaceutique topique selon la revendication 4, dans laquelle la concentration en kétoprofène, calculée en tant qu'acide libre, est comprise dans la plage allant de 2,5 % en poids à 4 % en poids lorsque c'est la forme racémique, et de 1,25 % en poids à 2 % en poids lorsque c'est l'isomère S(+).

6. Composition pharmaceutique topique selon la revendication 2, dans laquelle la concentration en filtre UV méthoxycinnamate d'octyle est comprise dans la plage allant de 0,5 % en poids à 5 % en poids.

7. Composition pharmaceutique topique selon la revendication 6, dans laquelle la concentration en filtre UV méthoxycinnamate d'octyle est comprise dans la plage allant de 1 % en poids à 3 % en poids.

8. Composition pharmaceutique topique selon la revendication 1, dans laquelle la concentration en filtre UV acide 2-phényl-benzimidazol-5-sulfonique est comprise dans la plage allant de 0,5 % en poids à 5 % en poids.

9. Composition pharmaceutique topique selon la revendication 8, dans laquelle la concentration en filtre UV acide 2-phényl-benzimidazol-5-sulfonique est comprise dans la plage allant de 1 % en poids à 3 % en poids.

10. Composition pharmaceutique topique selon la revendication 1, dans laquelle l'antioxydant est le butylhydroxytoluène (BHT) et sa concentration est comprise dans la plage allant de 0,01 % en poids à 0,2 % en poids.

11. Composition pharmaceutique topique selon la revendication 10, dans laquelle la concentration en antioxydant BHT est comprise dans la plage allant de 0,01 % en poids à 0,06 % en poids.

12. Composition pharmaceutique selon l'une quelconque des revendications 1 à 11, dans laquelle l'agent pour améliorer la perméabilité à travers la peau éventuellement utilisé est une substance choisie dans le groupe constitué par l'urée, la pyrrolidone, la N-méthylpyrrolidine, le décylméthylsulfoxyde, le laurylsulfate de sodium, et le diméthylsulfoxyde.

13. Composition pharmaceutique selon l'une quelconque des revendications 1 à 12, dans laquelle de l'huile de lavande est utilisée comme arôme.

14. Composition pharmaceutique selon l'une quelconque des revendications 1 à 13, sous la forme de crèmes, gels, lotions, sprays ou poudres, dans lesquelles l'agent anti-inflammatoire non stéroïdien est protégé de la photodégradation, et la composition n'a pas d'effets résiduaires photoxiques et photoallergiques suite à une application sur la peau.

15. Composition pharmaceutique topique selon la revendication 1, comprenant de 2,5 à 4 % en poids de kétoprofène sous la forme d'un mélange d'isomères ou de 1,25 à 2 % en poids sous la forme de l'isomère S(+) seul ; de 0,5 à 5 % en poids d'un agent gélifiant ; de 1 à 3 % en poids d'un premier filtre UV (de préférence un dérivé de l'acide cinnamique) ; de 1 à 3 % en poids d'un deuxième filtre UV (l'acide phénylbenzimidazolsulfonique) ; de 0 à 2 % en poids d'un conservateur ; de 0 à 20 % en poids d'un agent améliorant la perméabilité ; de 0,01 à 0,06 % en poids de BHT ; de 0 à 0,1 % en poids d'EDTA sodique ; de 0 à 1 % en poids d'arômes ; de 20 à 90 % en poids d'éthanol ; et de l'eau pour atteindre 100 % en poids.

16. Utilisation de méthoxycinnamate d'octyle en combinaison avec l'acide 2-phényl-benzimidazol-5-sulfonique et du BHT pour la préparation de compositions pharmaceutique selon la revendication 1, pour le traitement topique de pathologies inflammatoires et de douleurs.

17. Formulations pharmaceutiques selon la revendication 1, pour le traitement topique d'une douleur et d'une inflammation associée à des lésions du tissu mou, des entorses et des distorsions, des ecchymoses, une tendinite et une inflammation veineuse.
